(19) Europäisches Patentamt
European Patent Office
Office européen des brevets

(11) EP 4 260 845 A1

(12) **EUROPEAN PATENT APPLICATION**

(43) Date of publication:
**18.10.2023 Bulletin 2023/42**

(21) Application number: **23167362.5**

(22) Date of filing: **11.04.2023**

(51) International Patent Classification (IPC):
***A61K 9/10*** *(2006.01)* ***A61P 27/02*** *(2006.01)*
***A61K 47/10*** *(2017.01)*

(52) Cooperative Patent Classification (CPC):
**A61K 9/0048; A61K 9/10; A61K 31/00; A61K 47/10; A61K 47/183; A61K 47/44; A61P 27/02**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **13.04.2022 US 202263330324 P**

(71) Applicant: **Caravel Therapeutics, Inc.**
**Taipei City 100 (TW)**

(72) Inventors:
- **SU, Wei-Wen**
  **100 Taipei City (TW)**
- **LEE, Chien-Hung**
  **100 Taipei City (TW)**

(74) Representative: **2K Patentanwälte Blasberg Kewitz & Reichel**
**Partnerschaft mbB**
**Schumannstrasse 27**
**60325 Frankfurt am Main (DE)**

(54) **PRESERVATIVE-FREE OPHTHALMIC PHARMACEUTICAL EMULSION AND ITS APPLICATION**

(57) The present invention provides a preservative-free ophthalmic pharmaceutical emulsion containing a nitric oxide-donating prostaglandin F2$\alpha$ derivative or a pharmaceutically acceptable salts or esters thereof as an active ingredient. The present invention also provides its application of promoting blood velocity of optic nerve head (ONH) for treating glaucoma patients and lowering risks for open angle glaucoma and retinal ischemia disease, such as retinal vein occlusion (RVO), including central retinal vein occlusion (CRVO) and branch retinal vein occlusion (BRVO), and retinal artery occlusion (RAO), including central retinal artery occlusion (CRAO) and branch retinal artery occlusion (BRAO).

EP 4 260 845 A1

## Description

BACKGROUND OF THE INVENTION

1. Technical Field

The present invention relates to a preservative-free ophthalmic pharmaceutical emulsion containing a nitric oxide-donating prostaglandin F2α derivative or a pharmaceutically acceptable salts or esters thereof as an active ingredient and its application of promoting blood velocity of optic nerve head (ONH) or treating glaucoma patients or patients with eye disease associated with retinal ischemia disease, such as retinal artery occlusion (RAO), including central retinal artery occlusion (CRAO) and branch retinal artery occlusion (BRAO), and retinal vein occlusion (RVO), including central retinal vein occlusion (CRVO) and branch retinal vein occlusion (BRVO).

2. Description of Related Art

Open-angle glaucoma (OAG) is a leading cause of irreversible blindness characterized by progressive neural rim loss and visual field damage, and elevated intraocular pressure (IOP) is a major risk factor of this disease progression. Therefore, reducing the IOP can slow the progression of visual field loss in patients with OAG and also lower the risk of onset of OAG in patients with ocular hypertension (OHT). Making pharmacologic reduction of IOP becomes the first-line treatment in most patients with OAG or OHT.

Previous reports have identified compromised vascular endothelial cell function in patients with primary open-angle glaucoma (POAG) and normal tension glaucoma (NTG), their nitric oxide (NO) and endothelin-1 balance were different. Besides, the other previous reports showed that the NO concentration in aqueous humor was different between glaucoma patients and non-glaucoma patients. Therefore, NO not only participates in blood flow and IOP regulation, but also in glaucomatous ganglion cell apoptosis.

Latanoprostene bunod (LBN) is a nitric oxide-donating prostaglandin F2α analog rapidly metabolized into latanoprost acid and butanediol mononitrate in situ after administration to the eye. Latanoprost acid reduces IOP by increasing aqueous humor outflow primarily through the uveoscleral pathway. On the other hand, NO released from butanediol mononitrate relaxes trabecular meshwork and Schlemm's canal, causing increased aqueous humor outflow and reduction of IOP.

Several studies have shown the efficiency of the latanoprostene bunod ophthalmic solution 0.024% (LBN 0.024%) on reducing IOP, such as Araie M, et al. revealed that LBN 0.024% provided 24-hr reduction of IOP in healthy eyes in Japanese population (Japanese subjects. Adv Ther., 2015;32:1128-1139); Kawase K, et al. further obtained that the IOP lowering effect sustained through one year in patients with OAG or OHT (Adv Ther., 2016;33(9):1612-1627); Sforzolini B, et al. found that LBN 0.024% to be more effective than latanoprost 0.005% in reducing IOP in patients with OAG or OHT (Br J Ophthalmol., 2015;99:738-745); Liu JH, et al. demonstrated that LBN 0.024% reduced IOP more efficiently during nocturnal period compared to timolol maleate 0.5% in patients with OAG or OHT (Am J Ophthalmol., 2016;169:249-257); Weinreb RN, et al. and Medeiros FA, et al. showed significantly greater IOP lowering with LBN 0.024% than Timolol 0.5% in patients with OAG or OHT (Clin Ophthalmol., 2014;8:1097-1104; Am J Ophthalmol., 2016;168:250-259).

BRIEF SUMMARY OF THE INVENTION

Though lowering of IOP is the principle of glaucoma treatment, increasing evidences suggest that decreased blood flow in the optic nerve plays a role in the development and progression of glaucoma. Therefore, there is a need to improve ocular blood flow to treat those glaucoma patients. In addition, it is known that ophthalmic risk factors for RVO or RAO are ocular hypertension and glaucoma, lower ocular perfusion pressure, and congenital and acquired changes in retinal arteries. Therefore, controlling the development and progression of glaucoma can effectively lower the ophthalmic risk factors for retinal ischemia disease.

As described above, topical LBN is used for reducing IOP by increasing aqueous humor outflow. However, there is no previous documents describe that LBN promote blood velocity or blood flow of ONH. Accordingly, the present inventors made intensive studies in order to find a new pharmaceutical application of a prostaglandin F2α derivative, LBN. As a result, they found that LBN promote the blood velocity of ONH of the patients with glaucoma, which may prevent glaucoma progression. On the other hand, improved ocular blood flow may also reduce risk of RVO or RAO. In other words, topical administrating LBN can act directly on the ONH or posterior pole of the eye for patients with glaucoma and patients with RVO or RAO. Besides, the conventional compositions comprise preservatives, such as benzalkonium chloride (BAK) or benzyl alcohol, which may have toxic effects on the neurosensory retina. In this regard, the present invention excludes the use of preservatives, thereby enhancing the safety of medication use and the potential for clinical

application in injectable dosage forms.

An aspect of the present invention provides a preservative-free ophthalmic pharmaceutical emulsion, comprising: 0.01% w/v to 0.1% w/v latanoprostene bunod or a pharmaceutically acceptable salt thereof, 0.2% w/v to 0.7% w/v an oil, 0.4% w/v to 1.4% w/v a surfactant, and at least one pharmaceutical excipient, wherein the HLB values of the oil and the surfactant are calculated together to form a required hydrophilic-lipophilic balance (rHLB) value in a range from 10 to 16.

An another aspect of the present invention provides a method of preventing or treating retinal ischemia disease in a subject, comprising: administering an effective amount of a preservative-free ophthalmic pharmaceutical emulsion to eyes of the subject, wherein the preservative-free ophthalmic pharmaceutical emulsion comprises 0.01% w/v to 0.1% w/v latanoprostene bunod or a pharmaceutically acceptable salt thereof, 0.2% w/v to 0.7% w/v an oil, 0.4% w/v to 1.4% w/v a surfactant, and at least one pharmaceutical excipient, wherein the HLB values of the oil and the surfactant are calculated together to form a required hydrophilic-lipophilic balance (rHLB) value in a range from 10 to 16.

An another aspect of the present invention provides a method of treating a subject with open angle glaucoma, comprising: administering an effective amount of the preservative-free ophthalmic pharmaceutical emulsion to eyes of the subject, wherein the preservative-free ophthalmic pharmaceutical emulsion comprises 0.01% w/v to 0.1% w/v latanoprostene bunod or a pharmaceutically acceptable salt thereof, 0.2% w/v to 0.7% w/v an oil, 0.4% w/v to 1.4% w/v a surfactant, and at least one pharmaceutical excipient, wherein the HLB values of the oil and the surfactant are calculated together to form a required hydrophilic-lipophilic balance (rHLB) value in a range from 10 to 16.

In some embodiments, the oil is selected from the group consisting of castor oil, olive oil, and sesame oil.

In some embodiments, the surfactant is selected from the group consisting of sodium dodecyl sulphate (SDS), sodium lauryl sulphate (SLS), polyoxyethylene sorbitan fatty acid esters (Tweens), polyoxyethylene stearates, sorbitan fatty acid esters (Spans), and polyethylene glycol 15 hydroxystearate.

In some embodiments, the at least one pharmaceutical excipient is selected from the group consisting of a co-solvent, a chelating agent, a pH adjuster, and a solvent.

In some embodiments, the chelating agent is EDTA or a salt thereof.

In some embodiments, the co-solvent is propylene glycol in a range from 1.6% w/v to 2.0% w/v.

In some embodiments, the preservative-free ophthalmic pharmaceutical emulsion comprises 0.024% w/v to 0.04% w/v latanoprostene bunod or a pharmaceutically acceptable salt thereof, 0.5% w/v olive oil, 0.4% w/v to 1.0% w/v polyethylene glycol 15 hydroxystearate, 1.8% w/v propylene glycol, 0.5% w/v EDTA disodium, and water in a sufficient amount.

In some embodiments, the preservative-free ophthalmic pharmaceutical emulsion comprises 0.024% w/v to 0.04% w/v latanoprostene bunod or a pharmaceutically acceptable salt thereof, 0.7% w/v sesame oil, 0.4% w/v to 1.4% w/v polyethylene glycol 15 hydroxystearate, 2.0% w/v propylene glycol, 0.5% w/v EDTA disodium, and water in a sufficient amount.

In some embodiments, the preservative-free ophthalmic pharmaceutical emulsion comprises 0.024% w/v to 0.04% w/v latanoprostene bunod or a pharmaceutically acceptable salt thereof, 0.2% w/v to 0.6% w/v castor oil, 0.4% w/v polyethylene glycol 15 hydroxystearate, 1.6% w/v propylene glycol, 0.5% w/v EDTA disodium, and water in a sufficient amount.

In some embodiments, the preservative-free ophthalmic pharmaceutical emulsion has a particle size distribution of D10 in a range between 20 nm and 30 nm, D50 in a range between 45 nm and 80 nm, and D90 in a range between 125 nm and 500 nm within 2 months storage in 2-8°C.

In some embodiments, treating the subject with open angle glaucoma is to reduce intraocular pressure and increase blur rate of retinal vessel area of the subject.

In some embodiments, the preservative-free ophthalmic pharmaceutical emulsion is administered to the subject by contacting with eyes or injecting to eyes.

## BRIEF DESCRIPTION OF THE DRAWINGS

None.

## DETAILED DESCRIPTION OF THE INVENTION

In the following detailed description, for purposes of explanation, numerous specific details are set forth in order to provide a thorough understanding of the disclosed embodiments. It will be apparent, however, that one or more embodiments may be practiced without these specific details.

As used herein, the terms "comprising," "having," and "including" are used in their open, non-limiting sense. The terms "a," "an," and "the" are understood to encompass the plural as well as the singular. The expression "one or more" means "at least one" and thus may include an individual characteristic or mixtures/combinations.

The present invention provides an agent containing a nitric oxide-donating prostaglandin F2α derivative or a pharmaceutically acceptable salts or esters thereof as an active ingredient, wherein said nitric oxide-donating prostaglandin F2α derivative is preferably latanoprostene bunod (LBN). More specifically, the present invention provides a preservative-free ophthalmic pharmaceutical emulsion, comprising: 0.01% w/v to 0.1% w/v latanoprostene bunod or a pharmaceutically acceptable salt thereof, 0.2% w/v to 0.7% w/v an oil, 0.4% w/v to 1.4% w/v a surfactant, and at least one pharmaceutical excipient, wherein the HLB values of the oil and the surfactant are calculated together to form a required hydrophilic-lipophilic balance (rHLB) value in a range from 10 to 16.

As used herein, the term "preservative" refers to a substance or a chemical that is added to products to prevent decomposition by microbial growth or by undesirable chemical changes. The preservative exhibiting sufficient antimicrobial effect on bacteria and fungi has traditionally been used in ophthalmic compositions. The preservative in the present invention includes BAK or its analog and other quaternary ammonium salts such as benzethonium chloride, benzyl alcohol, busan, cetrimide, chlorhexidine, chlorobutanol, mercurial preservatives, or phenylmercuric nitrate, phenylmercuric acetate, thimerosal, phenylethyl alcohol and the like.

As used herein, the term "emulsion" refers to a homogenous mixture of two liquid phases which do not normally mix such as oil and water.

As used herein, the term "nitric oxide-donating prostaglandin F2α derivative" refers to a prostaglandin F2α-related compound derived from the skeleton of prostanoic acid (such as, but not limited to, latanoprost, bimatoprost, travoprost, and carboprost) and can be metabolized to release NO. Preferably, the prostaglandin F2α derivative preferably is LBN of the following compound (I):

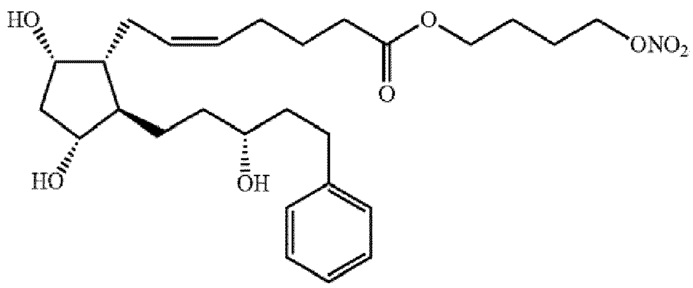

...(I).

As used herein, the term "pharmaceutically acceptable salts or esters" refers to pharmaceutically acceptable organic or inorganic salts and esters of a compound of the invention. Exemplary salts include, but not limited to, sulfate, citrate, acetate, oxalate, chloride, bromide, iodide, nitrate, bisulfate, phosphate, acid phosphate, isonicotinate, lactate, salicylate, acid citrate, tartrate, oleate, tannate, pantothenate, bitartrate, ascorbate, succinate, maleate, gentisinate, fumarate, gluconate, glucuronate, saccharate, formate, benzoate, glutamate, methanesulfonate mesylate, ethanesulfonate, benzenesulfonate, p-toluenesulfonate, and pamoate (i.e., 1,1-methylene-bis-(2-hydroxy- 3-naphthoate)) salts. Exemplary esters include, but not limited to, alkyl, alkenyl, alkynyl, aryl, arylalkyl, and cycloalkyl esters of acidic groups, including, but not limited to, carboxylic acids, phosphoric acids, phosphinic acids, sulfonic acids, sulfinic acids, boronic acids, nitric acid, and nitrous acid.

A pharmaceutically acceptable salts or esters may involve the inclusion of another molecule such as an acetate ion, a succinate ion or other counter ion. The counter ion may be any organic or inorganic moiety that stabilizes the charge on the parent compound. Furthermore, a pharmaceutically acceptable salts or esters may have more than one charged atom in its structure. Instances where multiple charged atoms are part of the pharmaceutically acceptable salts or esters may have multiple counter ions. Hence, a pharmaceutically acceptable salts or esters may have one or more charged atoms and/or one or more counter ion.

As used herein, the term "pharmaceutically acceptable" refers that the substance or composition must be compatible chemically and/or toxicologically, with the other ingredients comprising a formulation, and/or the mammal being treated therewith.

As used herein, the term "active ingredient" refers that the nitric oxide-donating prostaglandin F2α derivative or a pharmaceutically acceptable salt thereof causes or triggers the blood velocity of ONH flowing fast when applied to a subject such as human organism or an animal organism, preferably to a human organism.

As used herein, the term "hydrophilic-lipophilic balance (HLB)" refers to the balance of the size and strength of the hydrophilic and lipophilic moieties of a surfactant molecule. The HLB values are obtained based on prior arts, or it may be obtained by the following two calculation formulas.

For most polyhydric alcohol fatty acid esters, approximate values may be calculated with the formula:

$$HLB = 20x(1 - S/A)$$

wherein S refers to saponification number of the ester and A refers to acid number of the acid.

Many fatty acid esters do not give good saponification data. For these, a calculation may be based on the formula:

$$HLB = (E + P)/5$$

wherein E refers to weight percentage of oxyethylene content and P refers to weight percentage of polyhydric alcohol content.

In the present invention, the HLB values of the oil and the surfactant are calculated together to form a required hydrophilic-lipophilic balance (rHLB), which is formulated as follow:

$$rHLB = \frac{\underline{HLB^{O}} * \underline{W^{O}} + \underline{HLB^{S}} * \underline{W^{S}}}{\underline{W^{O}} + \underline{W^{S}}}$$

wherein the "HLBo" refers to the HLB value of the oil, and "Wo" refers to the weight of the oil (unit); wherein the "HLBs" refers to the HLB value of the surfactant, and "Ws" refers to the weight of the surfactant (unit).

In one or more embodiments, the amount of LBN in the present invention ranges from 0.001% w/v to about 1.0% w/v. For example, it can be in the range of any of the following values, like 0.001% w/v, 0.005 % w/v, 0.01% w/v, 0.015% w/v, 0.02% w/v, 0.025% w/v, 0.03% w/v, 0.035% w/v, 0.04% w/v, 0.045% w/v, 0.05% w/v, 0.055% w/v, 0.06% w/v, 0.065% w/v, 0.07% w/v, 0.075% w/v, 0.08% w/v,0.085% w/v, 0.09% w/v, 0.095% w/v, and 0.1% w/v. In a preferred embodiment, the amount of LBN ranges from about 0.01% w/v to about 0.1 w/v.

In one or more embodiments, the emulsion in the present invention further contains a pharmaceutically acceptable vehicle comprising at least one pharmaceutical excipient, such as oil, surfactants, emulsifiers, chelating agent, tonicity adjusting agents, buffering agents, pH adjusting agents, viscosity enhancers, co-solvent and other agents that may be used in formulating an ophthalmic composition. Preferably, the pharmaceutical excipient is a co-solvent, a chelating agent, a pH adjuster, a solvent, or the combination thereof.

Considering using the eye drops containing preservatives for a long-time may poison to ocular surface cells in mammals (including corneal epithelium and conjunctiva), and cause corneal epithelial cell death, corneal punctate ulcers, delayed corneal wound healing, conjunctival inflammation/subconjunctival fibrosis, tear film instability, dry eye symptoms, allergic reactions, and eye discomfort, etc., the present invention preferably excludes the addition of the preservatives.

The surfactants include, but not limited to, sodium dodecyl sulphate (SDS), sodium lauryl sulphate (SLS), polyoxyethylene sorbitan fatty acid esters (Tweens), polyoxyethylene stearates, sorbitan fatty acid esters (Spans), and polyethylene glycol 15 hydroxystearate. The surfactant in the present invention preferably is polyethylene glycol 15 hydroxystearate. The amount of the surfactant in the present invention preferably ranges from about 0.0005% w/v to about 5.0% w/v. For example, it can be in the range of any of the following values, like 0.0005% w/v, 0.001 % w/v, 0.005% w/v, 0.01% w/v, 0.05% w/v, 0.1% w/v, 0.3% w/v, 0.4% w/v, 0.5% w/v, 0.6% w/v, 0.7 % w/v, 0.8% w/v, 0.9% w/v, 1.0% w/v, 1.3% w/v, 1.4% w/v, 1.5% w/v, 1.6% w/v, 1.7% w/v, 1.9% w/v, 2.0% w/v, 2.3% w/v, 2.5% w/v, 2.7% w/v, 3.0% w/v, 3.2% w/v, 3.5% w/v, 3.8% w/v, 4.0% w/v, 4.1% w/v, 4.2% w/v, 4.3% w/v, 4.4% w/v, 4.5% w/v, 4.6% w/v, 4.7% w/v, 4.8% w/v, 4.9% w/v, and 5.0% w/v. In a preferred embodiment, the amount of the surfactant ranges from about 0.01% w/v to about 2.0% w/v, and most preferably from about 0.4% w/v to about 1.4% w/v.

The oil includes, but not limited to, castor oil, olive oil, and sesame oil. The amount of the oil in the present invention ranges from about 0.001% w/v to about 2.0% w/v. For example, it can be in the range of any of the following values, like 0.001% w/v, 0.005 % w/v, 0.01% w/v, 0.02% w/v, 0.03% w/v, 0.04% w/v, 0.05% w/v, 0.06% w/v, 0.07% w/v, 0.08% w/v, 0.09% w/v, 0.1% w/v, 0.2% w/v, 0.3% w/v, 0.4% w/v, 0.5% w/v, 0.6% w/v, 0.7% w/v, 0.8% w/v, 0.9% w/v, 1.0% w/v, 1.5% w/v, and 2.0% w/v. In a preferred embodiment, the amount of the oil ranges from about 0.02% w/v to about 1.0 w/v, and most preferably from about 0.2% w/v to about 0.7% w/v.

As used herein, the term "emulsifier" refers to a substance which aids the formation of an emulsion. The emulsifiers can be selected from the group consisting of non-ionic, cationic and anionic emulsifier. The amount of the emulsifier in the present invention ranges from about 0.001% w/v to about 5.0% w/v. For example, it can be in the range of any of the following values, like 0.001 % w/v, 0.005% w/v, 0.01% w/v, 0.05% w/v, 0.1% w/v, 0.2% w/v, 0.3% w/v, 0.5% w/v, 0.7 % w/v, 0.9% w/v, 1.0% w/v, 1.3% w/v, 1.5% w/v, 1.7% w/v, 2.0% w/v, 2.3% w/v, 2.5% w/v, 2.7% w/v, 3.0% w/v, 3.2% w/v, 3.5% w/v, 3.8% w/v, 4.0% w/v, 4.1% w/v, 4.2% w/v, 4.3% w/v, 4.4% w/v, 4.5% w/v, 4.6% w/v, 4.7% w/v, 4.8% w/v, 4.9% w/v, and 5.0% w/v. In a preferred embodiment, the amount of the emulsifier ranges from about 0.02% w/v to about 2.0% w/v, and more preferably from about 0.5% w/v to about 1.0% w/v.

Chelating agents can be selected from the group consisting of ethylenediaminetetraacetic acid (EDTA) or its salt, such as disodium edetate. The amount of the chelating agent in the present invention ranges from about 0.001% w/v to about 10% w/v, preferably about 0.1% w/v to about 3% w/v. For example, it can be in the range of any of the following values, like 0.001 % w/v, 0.005% w/v, 0.01% w/v, 0.05% w/v, 0.1% w/v, 0.5% w/v, 1.0% w/v, 1.5% w/v, 2.0% w/v, 2.5% w/v, 3.0% w/v, 3.5% w/v, 4.0% w/v, 4.5% w/v, 5.0% w/v, 5.5% w/v, 6.0% w/v, 6.5% w/v, 7.0% w/v, 7.5% w/v, 8.0% w/v, 8.5% w/v, 9.0% w/v, 9.5% w/v, and 10% w/v.

It is known that to be isotonic with the physiological fluids, a fluid has to have an osmolarity ranging from 225 to 375 mOsmol/L, particularly 300 mOsmol/L. Thus the pharmaceutically acceptable vehicle may contain tonicity adjusting agents.

In order to adjust the pH value of the emulsion in the range of about 5.0 to about 8.0, a pH adjusting agent may be contained in the pharmaceutically acceptable vehicle of the present invention. The pH value in the agent of the present invention can be about pH 5.0, about pH 5.5, about pH 6.0, about pH 6.5, about pH 7.0, about pH 7.5, and about pH 8.0.

The viscosity enhancers can increase the viscosity of the emulsion and provide a longer residence time in the eye, providing a longer time for drug absorption and effect. The pharmaceutically acceptable vehicle may contain the viscosity enhancers.

To enhance the surfactant effect, the co-solvent may be added in the emulsion. The co-solvent can be propylene glycol, polyethylene glycol, and glycerine. In one or more embodiments, the amounts of the co-solvent range from about 0.1% w/v to about 3% w/v. For example, it can be in the range of any of the following values, like 0.1% w/v, 0.2% w/v, 0.3% w/v, 0.4% w/v, 0.5% w/v, 0.6% w/v, 0.7 % w/v, 0.8% w/v, 0.9% w/v, 1.0% w/v, 1.2% w/v, 1.3% w/v, 1.4% w/v, 1.5% w/v, 1.6% w/v, 1.7% w/v, 1.8% w/v, 1.9% w/v, 2.0% w/v, 2.1% w/v, 2.2% w/v, 2.3% w/v, 2.4% w/v, 2.5% w/v, 2.6% w/v, 2.7% w/v, 2.8% w/v, 2.9% w/v, and 3% w/v. In a preferred embodiment, the amount of the co-solvent ranges from about 0.5% w/v to about 2.5% w/v, more preferably from about 1.6% w/v to about 2.0% w/v.

In one or more embodiments, the emulsion in the present invention is administered to the subject by contacting with eyes or injecting to eyes. Preferably, the mean of contacting with eyes is using an eye drop.

Specifically, the emulsion can be administered orally or parenterally. The dosage form for administration can include an eye drop, an ophthalmic ointment, an injection, a tablet, a capsule, a granule, a powder, and the like, and particularly preferred is an eye drop.

An eye drop, for example, can be prepared using a tonicity agent such as sodium chloride or concentrated glycerin, a buffer such as sodium phosphate or sodium acetate, a surfactant such as polyoxyethylene sorbitan monooleate, polyoxyl 40 stearate, polyoxyethylene hydrogenated castor oil or polyethylene glycol 15 hydroxystearate, a stabilizer such as sodium citrate or sodium edetate. The pH value of the eye drop is permitted as long as it falls within the range that is acceptable as an ophthalmic preparation. Preferred pH value ranges from 5 to 8, such as pH 5, 6, 7, and 8, especially pH 7.

An ophthalmic ointment can be prepared using a widely used base such as white soft paraffin or liquid paraffin according to need.

An oral preparation such as a tablet, a capsule, a granule or a powder can be prepared using an extender such as lactose, crystalline cellulose, starch or a vegetable oil, a lubricant such as magnesium stearate or talc, a binder such as hydroxypropyl cellulose or polyvinylpyrrolidone, a disintegrant such as carboxymethyl cellulose calcium or low-substituted hydroxypropylmethyl cellulose, a coating agent such as hydroxylpropylmethyl cellulose, macrogol or a silicone resin, a film forming agent such as gelatin film, or the like according to need.

The dose can be appropriately selected depending on the symptoms, age, dosage form and the like. An eye drop may be instilled once to several times a day at a concentration of from 0.00001% w/v to 1% w/v, preferably from 0.0001% w/v to 1% w/v. An oral preparation may be administered once or divided into several times at a dose of generally from 0.01 mg to 5000 mg per day, preferably from 0.1 mg to 1000 mg per day.

A preferred embodiment according to the invention is the preservative-free ophthalmic pharmaceutical emulsion, comprising: 0.024% w/v to 0.04% w/v latanoprostene bunod or a pharmaceutically acceptable salt thereof, 0.5% w/v olive oil, 0.4% w/v to 1.0% w/v polyethylene glycol 15 hydroxystearate, 1.8% w/v propylene glycol, 0.5% w/v EDTA disodium, and water in a sufficient amount.

A preferred embodiment according to the invention is the preservative-free ophthalmic pharmaceutical emulsion, comprising: 0.024% w/v to 0.04% w/v latanoprostene bunod or a pharmaceutically acceptable salt thereof, 0.7% w/v sesame oil, 0.4% w/v to 1.4% w/v polyethylene glycol 15 hydroxystearate, 2.0% w/v propylene glycol, 0.5% w/v EDTA disodium, and water in a sufficient amount.

A preferred embodiment according to the invention is the preservative-free ophthalmic pharmaceutical emulsion, comprising: 0.024% w/v to 0.04% w/v latanoprostene bunod or a pharmaceutically acceptable salt thereof, 0.2% w/v to 0.6% w/v castor oil, 0.4% w/v polyethylene glycol 15 hydroxystearate, 1.6% w/v propylene glycol, 0.5% w/v EDTA disodium, and water in a sufficient amount.

The sufficient amount of water means the residue of 100 parts deducted from the parts of the other components if the preservative-free ophthalmic pharmaceutical emulsion is in the amount of 100 parts by weight in total.

Preferably, the preservative-free ophthalmic pharmaceutical emulsion of the present invention has a particle size distribution of D10 in a range between 20 nm and 30 nm, D50 in a range between 45 nm and 80 nm, and D90 in a range between 125 nm and 500 nm within 2 months storage in 2-8°C.

As used herein, said D10, D50 and D90 are values typically used to characterize particle size distribution. Said values are derived from a particle size distribution, and they indicate the size below which a certain quantity of the sample lie; for instance, a particle size distribution of D10 in a range between 20 nm and 30 nm means that 10% of the sample is between 20 nm and 30 nm.

As used herein, "stability" is defined as the time during which pharmaceutical expertise or even raw material considered alone is maintained within specified limits and throughout the period of storage and use, the same conditions and characteristics that had upon the time of their manufacture. It can also be defined as the time period comprised between the time at which the product is being manufactured to that when its potency is reduced to not more than 10%, since the alteration products are all securely identified and previously recognized their effects.

Further, the present invention also provides an application of the agent containing a nitric oxide-donating prostaglandin F2$\alpha$ derivative or a pharmaceutically acceptable salts or esters thereof as an active ingredient for promoting blood velocity of optic nerve head (ONH) or for treating or preventing retinal ischemia diseases for those glaucoma patients. Specifically, the present invention provides a method of preventing or treating retinal ischemia disease in a subject, comprising: administering (contact with eyes or inject to eyes) an effective amount of the preservative-free ophthalmic pharmaceutical emulsion to eyes of the subject, wherein the preservative-free ophthalmic pharmaceutical emulsion comprises: 0.01% w/v to 0.1% w/v latanoprostene bunod or a pharmaceutically acceptable salt thereof, 0.2% w/v to 0.7% w/v an oil, 0.4% w/v to 1.4% w/v a surfactant, and at least one pharmaceutical excipient, and wherein the HLB values of the oil and the surfactant are calculated together to form a required hydrophilic-lipophilic balance (rHLB) value in a range from 10 to 16.

As used herein, the term "optic nerve head" refers to the circular area in the back (posterior segment) of the eye where the optic nerve connects to the retina. The optic nerve head encompasses the lamina cribosa.

As used herein, the term "retinal ischemia diseases" refers to an eye disease associated with retinal vascular disorder that affects the blood vessels of the eye. Diseases and conditions that affect the blood vessels in the eyes can lead to vision impairment and vision loss. Retinal ischemia diseases may be associated with other medical problems, such as hypertension (high blood pressure), atherosclerosis, and issues with blood circulation. Common types of retinal ischemia diseases includes, but not limited to, retinal artery occlusion (RAO), including central retinal artery occlusion (CRAO) and branch retinal artery occlusion (BRAO) or retinal vein occlusion (RVO), including central retinal vein occlusion (CRVO) and branch retinal vein occlusion (BRVO).

Further, the present invention also provides a method of treating a subject with open angle glaucoma; more specifically, said method reduces intraocular pressure and increases blur rate of retinal vessel area in a subject. The method comprises: administering (contact with eyes or inject to eyes) an effective amount of the preservative-free ophthalmic pharmaceutical emulsion to eyes of the patient, wherein the preservative-free ophthalmic pharmaceutical emulsion comprises: 0.01% w/v to 0.1% w/v latanoprostene bunod or a pharmaceutically acceptable salt thereof, 0.2% w/v to 0.7% w/v an oil, 0.4% w/v to 1.4% w/v a surfactant, and at least one pharmaceutical excipient, wherein the HLB values of the oil and the surfactant are calculated together to form a required hydrophilic-lipophilic balance (rHLB) value in a range from 10 to 16.

The invention will now be illustrated by the following description of clinical examples. Further characteristics of the invention will become clear from the following clinical observations.

EXAMPLES

***Formulation of Latanoprostene bunod***

Latanoprostene bunod and castor oil are taken and mixed as an oil phase mixture in a glass beaker, and the mixture is stirred continuously using a dry glass rod until complete solubilization of latanoprost takes place.

Polyethylene glycol 15 hydroxystearate is heated in a separate glass beaker at 65°C-70°C until it melts. After melting, the polyethylene glycol 15 hydroxystearate is transferred to the abovementioned oil phase mixture to form an oil phase solution, and is stirred using dry glass rod at 65°C to 70°C.

Till complete mixing, the temperature of the oil phase solution is allowed to cool down to 60°C with gentle stirring. Said temperature is maintained at around 55°C to 60°C, and then the oil phase solution is added dropwise under continuous stirring to an aqueous phase of water for injection at 55°C to 60°C, leading to the formation of a transparent micro-emulsion.

Next, the propylene glycol is added at 55°C to 60°C. The mixture is allowed to cool down to 25°C to 30°C with gentle stirring and keeps stirring for another 30 to 45 minutes at 25°C to 30°C.

Finally, the pH value of the mixture is checked and adjusted to 7.0 by adding HCl or NaOH solution. Its volume

is made up to 100% by rinsing the manufacturing vessels with WFI (water for injection) and the composition is aseptically filtered through 0.2 $\mu$m membrane filter. The emulsion is filled into naturally occurring low density polyethylene containers.

The ingredients and the HLB values of the exemplary emulsion are shown below:

Table 1

| **Example 1** | **Quantity (% w/v)** |
|---|---|
| Latanoprostene bunod | 0.04 |
| Propylene glycol | 1.6 |
| Polyethylene glycol 15 Hydroxy-stearate (HLB=16) | 0.4 |
| Castor oil (HLB=14) | 0.2 |
| Sodium Edetate (EDTA·2Na) | 0.5 |
| Sodium Hydroxide | Q.S. |
| Hydrochloric acid | Q.S. |
| Water for Injection (WFI) | Q.S. |

Table 2

| **Example 2** | **Quantity (% w/v)** |
|---|---|
| Latanoprostene bunod | 0.04 |
| Propylene glycol | 1.8 |
| Polyethylene glycol 15 Hydroxy-stearate (HLB=16) | 0.4 |
| Olive oil (HLB=7) | 0.5 |
| Sodium Edetate (EDTA·2Na) | 0.5 |
| Sodium Hydroxide | Q.S. |
| Hydrochloric acid | Q.S. |
| Water for Injection (WFI) | Q.S. |

Table 3

| **Example 3** | **Quantity (% w/v)** |
|---|---|
| Latanoprostene bunod | 0.04 |
| Propylene glycol | 2.0 |
| Polyethylene glycol 15 Hydroxy-stearate (HLB=16) | 0.4 |
| Sesame oil (HLB=7) | 0.7 |
| Sodium Edetate (EDTA·2Na) | 0.5 |
| Sodium Hydroxide | Q.S. |
| Hydrochloric acid | Q.S. |
| Water for Injection (WFI) | Q.S. |

***Stability study***

In this section, stability study is conducted using the abovementioned Example 1 as an example. The stability study is performed according to common practices known such as ICH HARMONISED TRIPARTITE GUIDELINE-Topic Q1A(R2) <Stability Testing of new Drug Substances and Products> and inspection items are screened based on USP

chapter 771 and 1771. The stability study is carried out under two conditions: temperature of 5°C±3°C with no humidity; and temperature of 25°C±2°C with the relative humidity of 60%±5%.

***Particle Size Distribution(PSD) testing***

a. Equipment: Nano Particle Size and Zeta-potential Analyzer (ELSZ-2000ZS)
b. Sample: 3 mL in a cuvette
c. Conditions are as follows:

| Item | Parameter | |
|---|---|---|
| Material | Refractive index | 1.3328 |
| | Viscosity | 0.8878 mPa s |
| Measurement | Temperature | 25°C |
| | Cumulative number | 100 |

The results of the stability study and PSD testing of Example 1 are shown in Table 4.

Table 4

| Condition | | 0M | Long-Term (5°C±3°C) | | Accelerated (25°C ± 2°C / 60 ± 5% RH) | |
|---|---|---|---|---|---|---|
| Time point | | | 1 Month | 2 Months | 1 Month | 2 Months |
| Appearance | | Translucent | Translucent | Translucent | Translucent | Translucent |
| Related substances (%) | Max. individual impurity | 0.071 | 0.073 | 0.075 | 0.082 | 0.079 |
| | Total impurities | 0.189 | 0.205 | 0.226 | 0.261 | 0.248 |
| pH | | 7.09 | 7.04 | 7.03 | 7.05 | 7.04 |
| Osmolality (mOsmol/kg) | | 267 | 268 | 269 | 268 | 269 |
| PSD (nm) | D,10 | 28.9 | 26.6 | 26.6 | 27.0 | 25.8 |
| | D,50 | 65.0 | 57.8 | 60.3 | 57.6 | 60.1 |
| | D,90 | 158.5 | 159.5 | 163.0 | 162.0 | 157.8 |
| Zeta potential (mV) | | 2.33 | 3.25 | 2.46 | 2.45 | 2.48 |

The study demonstrates that all tests based on the methodology at time 0, 1st, and 2nd months under the long-term condition and the accelerated condition are stable.

The results obtained during the long-term and accelerated stability study demonstrate that the preservative-free ophthalmic pharmaceutical emulsion is stable at both fridge and room temperature. The data indicates that the assay of LBN remained unchanged upon storage under the accelerated condition.

***HPLC method***

LBN content is determined with an HPLC equipped with an UV detector and using a mixture of two mobile phases. The HPLC condition reports as the following:

| | |
|---|---|
| Buffer | 0.1% Perchloric acid (70%) into 1000 mL $H_2O$ |
| Mobile phase A | Buffer: MeOH=80:20 |
| Mobile phase B | ACN:$H_2O$=90:10 |

(continued)

| | |
|---|---|
| Column | Chiralcel OJ-3R, 150*4.6 mm, 3 μm or an equivalent |
| | (Make: Diacel; P/N.: 17824) |
| Sample temp. | 8°C |
| Column temp. | 40°C |
| Flow rate | 1.0 mL/min |
| Injection volume | 40μl |
| Detector | UV 210 nm |
| Run time | 50 min |
| Post time | 10 min |

***rHLB range study***

a. Preparation: using various rHLB such as 16, 14.83, 12.49, 10.15, 8.98, 6.64
b. After compounding, appearance change as the following:

| Condition | rHLB range | Appearance | Note |
|---|---|---|---|
| Initial | 6.64~10.15 | Milky appearance | w/o phase separation |
| | 12.49~16 | Translucent appearance | w/o phase separation |
| Overnight at room temperature | 6.64~8.98 | Milky appearance | w/ phase separation |
| | 10.15 | Milky appearance | w/o phase separation |
| | 12.49~16 | Translucent appearance | w/o phase separation |

Note: rHLB 10-16: without phase separation even if storage overnight at room temperature.

***The subjects***

The patients diagnosed with open angle glaucoma in total are 35 eyes of 20 patients, wherein 10 eyes are diagnosed as POAG, and 25 eyes are NTG, besides 2 eyes had received prior corneal refractive surgery, and 1 eye had undergone cataract extraction and intraocular lens implantation. In the aspect of the medication using, 20 eyes are treatment naïve upon entry of the study (that is they are not using IOP lowering medication), and 15 eyes are previously treated with IOP lowering medication. The IOP lowering medication in the treated patients were requested for the following medication washout period: 4 weeks for beta-blockers and prostaglandin analogues, 2-4 weeks for alpha-agonists, and 1 week for carbonic anhydrase inhibitors in order to eliminate the experimental errors.

The demographic, clinical and ocular characteristics of the whole patients are listed below.

Table 5

| | |
|---|---|
| **Demographic characteristics** | |
| Number of patients | 20 |
| Number of disease eyes | 35 |
| Age at diagnosis | 53.3 ± 12.98 |
| Male | 6 (30%) |
| **Clinical characteristics** | |
| Number of treatment naive eyes | 20 |
| Pretreatment glaucoma mediation | 15 |
| Number of eyes using prostaglandin analog | 3 |

(continued)

| Clinical characteristics | |
|---|---|
| Number of eyes using beta blockers | 8 |
| Number of eyes using alpha-2-sympathomimetics | 5 |
| Number of eyes using carbonic anhydrase inhibitors | 1 |
| Number of eyes with previous ocular surgery | 3* |
| **Ocular characteristics** | |
| Number of eyes diagnosed as POAG/NTG | 10/25 |
| Mean IOP in the diseased eye at diagnosis (mmHg) | 17.68 ± 4.11 |
| Mean CCT (μm) | 537.97 ± 40.17 |
| Mean RNFL thickness (μm) | 75.94 ± 15.36 |
| MD (dB) | -4.90± 5.77 |

Note: IOP is intraocular pressure. POAG is primary open-angle glaucoma. NTG is normal tension glaucoma. CCT is central corneal thickness. RFNL is retinal nerve fiber layer. MD is mean deviation.

Note: The symbol "*" means 2 eyes received laser-assisted in situ keratomileusis, and 1 eye received cataract extraction and intraocular lens implantation.

***Testing method***

All patients are received the best corrected visual acuity test (BCVA), IOP measurement, optical coherence tomography (OCT), laser speckle flowgraph (LSFG) evaluation, fundus photo, visual field examination, blood pressure (BP), and heart rate measurement, and then these obtained data are averaged as the baseline data of the experiment, but the intraocular eye disease other than OAG, significant cataract influencing refractive errors or visual field result, systemic disease with resultant visual field defect, and unreliable LSFG results are excluded.

In the LSFG evaluation, one drop of said emulsion is instilled in the examined eyes at clinic. Afterward, LSFG measurements are repeated at 30 minutes and 2 hours post-LBN instillation. The patients would continuously instill the emulsion once every night for one month, and return to clinic again for another LSFG exam. Laser speckle flowgraph (LSFG-NAVI version 3.1.39.2 software, Softcare Ltd., Fukuoka, Japan) are used to measure the mean blur rate (MBR) of the optic nerve head area for pulse waveform analysis in all patients. Averaged MBR and pulse waveform parameters are documented at each LSFG measurement in order to evaluate the effect of latanoprostene bunod using on microcirculation of optic nerve head.

Visual field tests are performed using the Humphrey Field Analyzer (Carl Zeiss Meditec Inc, Dublin, California, USA) with a 30-2 test pattern, size III white stimulus Swedish interactive threshold algorithm (SITA) standard program.

***Results***

According to Table 6, the mean intraocular pressure (MIOP) at baseline, 30 minutes, 2 hours and 1 month were 17.68 ± 4.11, 18.29 ± 4.00, 17.24 ± 4.14 and 12.02 ± 5.31 mmHg respectively. The MIOPs have not differed at 30 minutes and 2 hours interval from baseline, but the MIOP significantly reduces after 1 month of LBN using ($p < 0.05$).

The mean blood pressure (MBP) and mean ocular perfusion pressure (MOPP) are not significantly different.

The mean heart rate (MHR) is significantly lower at 30 minutes after the first LBN instillation ($p< 0.05$).

Table 6

| | Baseline | 30 minutes | 2 hours | 1 month |
|---|---|---|---|---|
| MIOP (mmHg) | 17.68 ± 4.11 | 18.29 ± 4.00 | 17.24 ± 4.14 | 12.92 ± 5.31* |
| MBP (mmHg) | 99.69 ± 9.90 | 99.62 ± 11.54 | 98.88 ± 14.12 | 98.08 ± 12.06 |
| MOPP (mmHg) | 48.82 ± 7.10 | 47.87 ± 8.07 | 48.65 ± 9.48 | 50.80 ± 12.06 |
| MHR (/minute) | 74.03 ± 8.95 | 69.05 ± 9.02* | 77.77 ± 11.11 | 72.03 ± 10.68 |

Note: The continuous variables are expressed as the mean ± SD. Differences in value among baseline and various post-treatment time are compared using Wilcoxon signed rank test. The symbol "*" means the $p < 0.05$, indicating statistical significance.

Note: MIOP is mean intraocular pressure. MBP is mean blood pressure. MOPP is mean ocular perfusion pressure. MHR is mean heart rate.

According to Table 7, the mean blur rate of vessel area (MBRv) increased after emulsion instillation. The difference reaches statistical significance at 2 hours and 1 month ($p < 0.05$). The percentage of MBRv (%) at 2 hours and 1 month are 114.36 ± 20.02 % and 113.91 ± 26.53 % respectively. The mean blur rate of tissue area (MBRt) is not significantly different.

Table 7

| | Baseline | 30 minutes | 2 hours | 1 month |
|---|---|---|---|---|
| MBRv (AU) | 40.24 ± 10.65 | 41.95 ± 10.03 | 45.14 ± 10.59* | 45.49 ± 12.22* |
| MBRv (%) | 100.0 ± 0 | 105.97 ± 16.49 | 114.36 ± 20.2* | 113.91 ± 26.53* |
| MBRt (AU) | 12.65 ± 3.57 | 12.48 ± 3.04 | 12.73 ± 3.46 | 12.67 ± 3.80 |
| MBRt (%) | 100.0 ± 0 | 100.63 ± 14.37 | 101.84 ± 13.71 | 101.84 ± 15.33 |

Note: The continuous variables were expressed as the mean ± SD. Differences in value among baseline and various post-treatment time were compared using Wilcoxon signed rank test. The symbol "*" means the $p < 0.05$, indicating statistical significance.

Note: MBRv is mean blur rate of vessel area. MBRt is mean blur rate of tissue area.

According to Table 8, the mean acceleration time index (MATI) at baseline, 30 minutes, 2 hours and 1 month are 32.62 ± 5.80, 31.51 ± 4.04, 30.51 ± 3.88, 31.71 ± 4.11 respectively. The MATI at 2 hours is significantly lower ($p= 0.01$). Other pulse parameters have not differed overtime.

Table 8

| | Baseline | 30 minutes | 2 hours | 1 month |
|---|---|---|---|---|
| MBOT (AU) | 50.53 ± 8.53 | 49.76 ± 6.17 | 52.09 ± 5.56 | 52.67 ± 6.62 |
| MBOS (AU) | 75.27 ± 6.51 | 74.44 ± 7.60 | 76.24 ± 6.67 | 77.26 ± 6.26 |
| MS (AU) | 11.92 ± 3.94 | 11.84 ± 2.08 | 11.82 ± 2.58 | 10.99 ± 2.30 |
| MATI (%) | 32.62 ± 5.80 | 31.51 ± 4.04 | 30.51 ± 3.88* | 31.71 ± 4.11 |
| MF (AU) | 13.76 ± 3.79 | 13.94 ± 4.30 | 13.46 ± 3.95 | 13.44 ± 3.31 |

Note: The continuous variables were expressed as the mean ± SD. Differences in value among baseline and various post-treatment time were compared using Wilcoxon signed rank test. The symbol "*" means the $p < 0.05$, indicating statistical significance.

Note: MBOT is mean blowout time. MBOS is mean blowout score. MS is mean skew. MATI is mean acceleration time index. MF is mean fluctuation.

The above experiment showed that relative blood velocity around ONH area significantly increases at 2 hours and 1 month after LBN instillation, reaching 114.36 ± 20.02 % and 113.91 ± 26.53 % from baseline respectively. Meanwhile, though the mean IOP significantly reduces at 1 month after LBN using, the mean ocular perfusion pressure has not differed at all time point of measurement.

In summary, the present invention provides a preservative-free and stable ophthalmic pharmaceutical emulsion containing a nitric oxide-donating prostaglandin F2$\alpha$ derivative or a pharmaceutically acceptable salts or esters thereof as an active ingredient, thereby preventing the destruction of the normal ocular surface tissue causing by the preservatives. Besides, the present invention provides a new application of the nitric oxide-donating prostaglandin F2$\alpha$ derivative or a pharmaceutically acceptable salts or esters thereof which can promote blood velocity of ONH, so that can treat or prevent open angle glaucoma and retinal ischemia disease.

The above is the detailed description of the present invention. However, the above is merely the preferred embodiment of the present invention and cannot be the limitation to the implement scope of the invention, which means the variation and modification according to the present invention may still fall into the scope of the present invention.

## Claims

**1.** A preservative-free ophthalmic pharmaceutical emulsion, comprising:

0.01% w/v to 0.1% w/v latanoprostene bunod or a pharmaceutically acceptable salt thereof,
0.2% w/v to 0.7% w/v an oil,
0.4% w/v to 1.4% w/v a surfactant, and
at least one pharmaceutical excipient,
wherein the HLB values of the oil and the surfactant are calculated together to form a required hydrophilic-lipophilic balance (rHLB) value in a range from 10 to 16.

**2.** The preservative-free ophthalmic pharmaceutical emulsion of claim 1, wherein:

the oil is selected from the group consisting of castor oil, olive oil, and sesame oil;
the surfactant is selected from the group consisting of sodium dodecyl sulphate (SDS), sodium lauryl sulphate (SLS), polyoxyethylene sorbitan fatty acid esters (Tweens), polyoxyethylene stearates, sorbitan fatty acid esters (Spans), and polyethylene glycol 15 hydroxystearate;
the at least one pharmaceutical excipient is selected from the group consisting of a co-solvent, a chelating agent, a pH adjuster, and a solvent, and preferably the chelating agent is EDTA or a salt thereof.

**3.** The preservative-free ophthalmic pharmaceutical emulsion of claim 1 or 2, wherein the co-solvent is propylene glycol in a range from 1.6% w/v to 2.0% w/v.

**4.** The preservative-free ophthalmic pharmaceutical emulsion of claim 1 or 2, comprising:

0.024% w/v to 0.04% w/v latanoprostene bunod or a pharmaceutically acceptable salt thereof,
0.5% w/v olive oil,
0.4% w/v to 1.0% w/v polyethylene glycol 15 hydroxystearate,
1.8% w/v propylene glycol,
0.5% w/v EDTA disodium, and
water in a sufficient amount.

**5.** The preservative-free ophthalmic pharmaceutical emulsion of claim 1 or 2, comprising:

0.024% w/v to 0.04% w/v latanoprostene bunod or a pharmaceutically acceptable salt thereof,
0.7% w/v sesame oil,
0.4% w/v to 1.4% w/v polyethylene glycol 15 hydroxystearate,
2.0% w/v propylene glycol,
0.5% w/v EDTA disodium, and
water in a sufficient amount.

**6.** The preservative-free ophthalmic pharmaceutical emulsion of claim 1 or 2, comprising:

0.024% w/v to 0.04% w/v latanoprostene bunod or a pharmaceutically acceptable salt thereof,
0.2% w/v to 0.6% w/v castor oil,
0.4% w/v polyethylene glycol 15 hydroxystearate,
1.6% w/v propylene glycol,
0.5% w/v EDTA disodium, and
water in a sufficient amount.

**7.** The preservative-free ophthalmic pharmaceutical emulsion of any one of claims 1 to 6, which has a particle size distribution of D10 in a range between 20 nm and 30 nm, D50 in a range between 45 nm and 80 nm, and D90 in a range between 125 nm and 500 nm within 2 months storage in 2-8°C.

**8.** A method of preventing or treating retinal ischemia disease in a subject, comprising:

administering an effective amount of a preservative-free ophthalmic pharmaceutical emulsion to eyes of the subject,

wherein the preservative-free ophthalmic pharmaceutical emulsion comprises 0.01% w/v to 0.1% w/v latanoprostene bunod or a pharmaceutically acceptable salt thereof,
0.2% w/v to 0.7% w/v an oil,
0.4% w/v to 1.4% w/v a surfactant, and
at least one pharmaceutical excipient,
wherein the HLB values of the oil and the surfactant are calculated together to form a required hydrophilic-lipophilic balance (rHLB) value in a range from 10 to 16.

**9.** The method of claim 8, wherein:

the oil is selected from the group consisting of castor oil, olive oil, and sesame oil;
the surfactant is selected from the group consisting of sodium dodecyl sulphate (SDS), sodium lauryl sulphate (SLS), polyoxyethylene sorbitan fatty acid esters (Tweens), polyoxyethylene stearates, sorbitan fatty acid esters (Spans), and polyethylene glycol 15 hydroxystearate;
the at least one pharmaceutical excipient is selected from the group consisting of a co-solvent, a chelating agent, a pH adjuster, and a solvent.

**10.** The method of claim 9, wherein the chelating agent is EDTA or a salt thereof.

**11.** The method of claim 9 or 10, wherein the preservative-free ophthalmic pharmaceutical emulsion is administered to the subject by contacting with eyes or injecting to eyes.

**12.** A method of treating a subject with open angle glaucoma, comprising:

administering an effective amount of a preservative-free ophthalmic pharmaceutical emulsion to eyes of the subject,
wherein the preservative-free ophthalmic pharmaceutical emulsion comprises:
0.01% w/v to 0.1% w/v latanoprostene bunod or a pharmaceutically acceptable salt thereof,
0.2% w/v to 0.7% w/v an oil,
0.4% w/v to 1.4% w/v a surfactant, and
at least one pharmaceutical excipient,
wherein the HLB values of the oil and the surfactant are calculated together to form a required hydrophilic-lipophilic balance (rHLB) value in a range from 10 to 16.

**13.** The method of claim 12, wherein the oil is selected from the group consisting of castor oil, olive oil, and sesame oil.

**14.** The method of claim 12 or 13, wherein treating the subject with open angle glaucoma is to reduce intraocular pressure and increase blur rate of retinal vessel area of the subj ect.

**15.** The method of any one of claims 12 to 14, wherein the preservative-free ophthalmic pharmaceutical emulsion is administered to the subject by contacting with eyes or injecting to eyes.

Europäisches Patentamt
European Patent Office
Office européen des brevets

# EUROPEAN SEARCH REPORT

Application Number
EP 23 16 7362

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2018/087092 A1 (BAUSCH & LOMB [US]; NICOX SA [FR]) 17 May 2018 (2018-05-17)<br>* page 1, line 21 - page 2, line 2 *<br>* page 16, line 1 - line 9 *<br>* page 4, line 15 - line 17 *<br>----- | 1-15 | INV.<br>A61K9/10<br>A61P27/02<br>A61K47/10 |
| Y | CN 109 172 580 A (ZHONGSHAN WANHAN PHARMACY CO LTD)<br>11 January 2019 (2019-01-11)<br>* example 6 *<br>----- | 1-15 | |
| Y | EP 3 643 702 A1 (NICOX SA [FR])<br>29 April 2020 (2020-04-29)<br>* example 16 *<br>----- | 1-15 | |
| Y | WO 2020/250252 A1 (SIFI SPA [IT]; CONSIGLIO NAZIONALE RICERCHE [IT])<br>17 December 2020 (2020-12-17)<br>* the whole document *<br>----- | 1-15 | |
| Y | WO 2019/045994 A1 (RHODES TECH [US])<br>7 March 2019 (2019-03-07)<br>* the whole document *<br>----- | 1-15 | TECHNICAL FIELDS SEARCHED (IPC)<br>A61K<br>A61P |
| A | EP 2 016 936 A1 (NOVAGALI PHARMA SA [FR])<br>21 January 2009 (2009-01-21)<br>* the whole document *<br>----- | 1-15 | |

The present search report has been drawn up for all claims

1

| Place of search | Date of completion of the search | Examiner |
|---|---|---|
| Munich | 11 September 2023 | Giró, Annalisa |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..........................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 23 16 7362

This annex lists the patent family members relating to the patent documents cited in the above-mentioned European search report.
The members are as contained in the European Patent Office EDP file on
The European Patent Office is in no way liable for these particulars which are merely given for the purpose of information.

11-09-2023

| Patent document cited in search report | Publication date | Patent family member(s) | Publication date |
|---|---|---|---|
| WO 2018087092 A1 | 17-05-2018 | BR 112019009330 A2 | 30-07-2019 |
| | | CA 3043000 A1 | 17-05-2018 |
| | | JP 7055802 B2 | 18-04-2022 |
| | | JP 2019534296 A | 28-11-2019 |
| | | KR 20190104993 A | 11-09-2019 |
| | | US 2020179401 A1 | 11-06-2020 |
| | | WO 2018087092 A1 | 17-05-2018 |
| CN 109172580 A | 11-01-2019 | NONE | |
| EP 3643702 A1 | 29-04-2020 | AR 047081 A1 | 04-01-2006 |
| | | AR 098931 A2 | 22-06-2016 |
| | | AU 2004313688 A1 | 28-07-2005 |
| | | BR PI0418245 A | 17-04-2007 |
| | | CA 2551409 A1 | 28-07-2005 |
| | | CN 1906159 A | 31-01-2007 |
| | | CR 8498 A | 15-11-2006 |
| | | CR 20110131 A | 30-03-2011 |
| | | CY 1117417 T1 | 26-04-2017 |
| | | DK 1704141 T3 | 02-05-2016 |
| | | EA 200601099 A1 | 27-10-2006 |
| | | EC SP066684 A | 25-10-2006 |
| | | EP 1704141 A1 | 27-09-2006 |
| | | EP 3002277 A1 | 06-04-2016 |
| | | EP 3643702 A1 | 29-04-2020 |
| | | ES 2566800 T3 | 15-04-2016 |
| | | ES 2767136 T3 | 16-06-2020 |
| | | GE P20094780 B | 25-09-2009 |
| | | GT 200500293 A | 06-07-2007 |
| | | HK 1096084 A1 | 25-05-2007 |
| | | HR P20160500 T1 | 03-06-2016 |
| | | HU E027357 T2 | 28-09-2016 |
| | | IL 176416 A | 30-05-2013 |
| | | JP 3984283 B2 | 03-10-2007 |
| | | JP 2007518716 A | 12-07-2007 |
| | | KR 20060113753 A | 02-11-2006 |
| | | KR 20080007415 A | 18-01-2008 |
| | | MA 28284 A1 | 01-11-2006 |
| | | ME 02433 B | 20-09-2016 |
| | | MX PA06007678 A | 01-09-2006 |
| | | MY 147181 A | 14-11-2012 |
| | | NO 337217 B1 | 15-02-2016 |
| | | NO 339685 B1 | 23-01-2017 |
| | | NZ 548271 A | 29-01-2010 |
| | | OA 13356 A | 13-04-2007 |
| | | PA 8620901 A1 | 30-08-2005 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO.

EP 23 16 7362

This annex lists the patent family members relating to the patent documents cited in the above-mentioned European search report. The members are as contained in the European Patent Office EDP file on The European Patent Office is in no way liable for these particulars which are merely given for the purpose of information.

11-09-2023

| Patent document cited in search report | Publication date | Patent family member(s) | Publication date |
|---|---|---|---|
| | | PE 20051052 A1 | 17-12-2005 |
| | | PL 1704141 T3 | 31-08-2016 |
| | | SI 1704141 T1 | 29-04-2016 |
| | | TN SN06210 A1 | 03-12-2007 |
| | | TW I337994 B | 01-03-2011 |
| | | UA 84726 C2 | 25-11-2008 |
| | | US 2005272743 A1 | 08-12-2005 |
| | | US 2008058392 A1 | 06-03-2008 |
| | | US 2009030076 A1 | 29-01-2009 |
| | | US 2010130507 A1 | 27-05-2010 |
| | | US 2011136904 A1 | 09-06-2011 |
| | | UY 28709 A1 | 31-08-2005 |
| | | WO 2005068421 A1 | 28-07-2005 |
| | | ZA 200605354 B | 30-09-2009 |
| WO 2020250252 A1 | 17-12-2020 | CN 114245737 A | 25-03-2022 |
| | | EP 3982930 A1 | 20-04-2022 |
| | | JP 2022541874 A | 28-09-2022 |
| | | US 2020390699 A1 | 17-12-2020 |
| | | US 2021205217 A1 | 08-07-2021 |
| | | WO 2020250252 A1 | 17-12-2020 |
| WO 2019045994 A1 | 07-03-2019 | AU 2018323861 A1 | 19-03-2020 |
| | | CA 3072768 A1 | 07-03-2019 |
| | | CN 111587111 A | 25-08-2020 |
| | | EP 3672587 A1 | 01-07-2020 |
| | | IL 272601 A | 31-03-2020 |
| | | KR 20200044874 A | 29-04-2020 |
| | | US 2021299046 A1 | 30-09-2021 |
| | | WO 2019045994 A1 | 07-03-2019 |
| EP 2016936 A1 | 21-01-2009 | AT 481087 T | 15-10-2010 |
| | | EP 2016936 A1 | 21-01-2009 |
| | | ES 2352296 T3 | 17-02-2011 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- Japanese subjects. *Adv Ther.,* 2015, vol. 32, 1128-1139
- *Adv Ther.,* 2016, vol. 33 (9), 1612-1627
- *Br J Ophthalmol.,* 2015, vol. 99, 738-745
- *Am J Ophthalmol.,* 2016, vol. 169, 249-257
- *Clin Ophthalmol.,* 2014, vol. 8, 1097-1104
- *Am J Ophthalmol.,* 2016, vol. 168, 250-259